# DEMANDE DE BREVET EUROPEEN

(11) **EP 1 642 570 A1**
(43) Date de publication de la demande: **05.04.2006**
(21) Numéro de dépôt: 05300790.2
(22) Date de dépôt: 03.10.2005
(51) Int. Cl.: A61K 8/99, A61Q 19/00, A61K 35/74, A23L 1/30, A23K 1/00

(54) **Composition cosmétique et/ou dermatologique pour peaux sensibles**

(30) Priorité: 04.10.2004 FR 0452258; 08.06.2005 EP 05012301
(71) Demandeur: L'OREAL, 75008 Paris (FR); NESTEC S.A., 1800 Vevey (CH)
(72) Inventeur: Gueniche, Audrey, 92500 Rueil Malmaison (FR); Breton, Lionel, 78000 Versailles (FR); Ballevre, Olivier, 200336 Shanghai (CN); Blum-Sperisen, Stéphanie, CH-1801 Mont-Pèlerin (CH); Bureau-Franz, Isabelle, CH-1110 Morges (CH); Benyacoub, Jalil, CH-1010 Lausanne (CH)
(74) Mandataire: Le Coupanec, Pascale A.M.P.

(57) **Abrégé**

La présente invention concerne l'utilisation d'une quantité efficace d'au moins un microorganisme appartenant à Pespèce *Lactobacillus paracaseï* ou *caseï,* l'une de leurs fractions ou l'un de leurs métabolites en association à une quantité efficace d'au moins un microorganisme appartenant à l'espèce *Bifidobacterium longum* ou *Bifidobacterium lactis,* l'une de leurs fractions ou l'un de leurs métabolites, pour la fabrication d'une composition dermatologique destinée à traiter et/ou prévenir les peaux sensibles réactives associées ou non à une sécheresse cutanée.

## Description

La présente invention concerne à titre principal l'utilisation d'association de microorganismes spécifiques pour la préparation dune composition, notamment cosmétique et/ou dermatologique destinée plus particulièrement à la prévention et/ou au traitement des peaux qualifiées de peaux sensibles et/ou sèches. Elle vise également des compositions correspondantes.

D'une manière générale, les peaux sensibles se définissent par une réactivité particulière de la peau. Toutefois, par opposition aux peaux qualifiées d'allergiques, cette réactivité ne relève pas d'un processus immunologique c'est-à-dire ne se produit pas uniquement au niveau dune peau déjà sensibilisée, en réponse à la présence d'un allergène. Son mécanisme est dit aspécifique.

Cette réactivité cutanée se traduit généralement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultra-violets ou infrarouges. Il existe également des facteurs associés comme l'âge et le type de peau. Ainsi les peaux sensibles sont plus fréquentes parmi les peaux sèches ou grasses que parmi les peaux normales.

L'apparition de ces signes d'inconfort, qui apparaissent dans les minutes qui suivent la mise en contact du sujet avec l'élément déclenchant, est une des caractéristiques essentielles des peaux sensibles. Il s'agit pour l'essentiel de sensations dysesthésiques. On entend par sensations dysesthésiques, des sensations plus ou moins douloureuses ressenties dans une zone cutanée comme les picotements, fourmillements, démangeaisons ou prurits, brûlures, échauffements, inconforts, tiraillements, etc. Ces signes subjectifs existent le plus souvent en l'absence de signes chimiques visibles tels que la rougeur et les desquamations. On sait aujourd'hui que ces réactions d'irritation et d'intolérance cutanée sont notamment liées à une libération de neuropeptides par les terminaisons nerveuses de l'épiderme et du derme.

Toutefois, on ne dispose toujours pas à ce jour de solution totalement satisfaisante pour prévenir et/ou traiter ce type de peaux qualifiées de sensible et ce problème est plus particulièrement exacerbé dans le cas où cette peau sensible est associée à une peau sèche. La peau sèche se manifeste essentiellement par une sensation de tiraillement et/ou de tension et elle est souvent associée à une baisse du taux d'hydratation cutanée et une altération de la fonction barrière, mesurée par la perte insensible en eau.

Le document WO 02/28402 décrit que des microorganismes probiotiques peuvent avoir un effet bénéfique dans la régulation de réactions d'hypersensibilité cutanée comme les réactions inflammatoires et allergiques qui relèvent d'un processus immunologique par opposition à la réactivité d'une peau sensible. Il est également fait état dans "Probiotics in the managment of atopic eczema, Clinical and Experimental Allergy 2000", Volume 30, pages 1604-1610, une étude rapportant l'effet individuel de quelques probiotiques sur les mécanismes immunitaires infantiles comme par exemple la dermatite atopique.

De manière inattendue, les inventeurs ont constaté qu'une association d'au moins deux microorganismes probiotiques spécifiques s'avérait tout particulièrement efficace, en particulier chez l'adulte, pour le traitement des peaux sensibles notamment associées à une peau sèche. L'association de microorganismes, considérée selon l'invention, manifeste avantageusement une activité potentielle au niveau de la barrière cutanée et une valence particulière dans le maintien des mécanismes de défense favorisant ainsi le maintien de l'homéostasie cutanée et la régulation du système immunitaire de la peau.

La présente invention concerne, selon un premier de ses aspects, l'utilisation d'une quantité efficace d'au moins un microorganisme appartenant à l'espèce *Lactobacillus paracaseï* ou *caseï,* l'une de leurs fractions ou l'un de leurs métabolites en association à une quantité efficace d'au moins un microorganisme appartenant à l'espèce *Bifidobacterium longum ou Bifidobacterium lactis,* l'une de leurs fractions ou l'un de leurs métabolites pour la préparation d'une composition utile pour prévenir et/ou traiter les peaux sensibles et/ou sèches.

En conséquence, la présente invention concerne en outre selon un autre de ses aspects, un procédé de traitement cosmétique pour prévenir et/ou traiter les peaux sensibles et/ou sèches, comprenant l'administration, notamment orale ou topique, d'une quantité efficace d'au moins un microorganisme appartenant à l'espèce *Lactobacillus paracaseï ou caseï,* l'une de leurs fractions ou l'un de leurs métabolites en association à une quantité efficace d'au moins un microorganisme appartenant à l'espèce *Bifidobacterium longum ou Bifidobacterium lactis,* l'une de leurs fractions ou l'un de leurs métabolites.

Selon une première variante, cette association est formulée sous la forme d'une composition pour l'absorption orale. Il peut notamment s'agir d'un complément alimentaire voire d'une denrée alimentaire.

Selon une seconde variante, elle se présente sous la forme d'une composition cosmétique et/ou dermatologique selon l'invention.

La présente invention concerne également selon un autre de ses aspects, une composition cosmétique et/ou dermatologique, notamment utile pour prévenir et/ou traiter les peaux sensibles et/ou sèches, comprenant dans un support physiologiquement acceptable une quantité efficace d'au moins un microorganisme appartenant à l'espèce *Lactobacillus paracaseï* ST11 NCC 2461 (CNCM I-2116), l'une de ses fractions ou l'un de ses métabolites en association à une quantité efficace d'au moins un microorganisme appartenant à l'espèce *Bifidobacterium longum* ou *Bifidobacterium lactis,* l'une de leurs fractions ou l'un de leurs métabolites.

La présente invention concerne, selon un autre de ses aspects, une composition cosmétique et/ou dermatologique, notamment utile pour prévenir et/ou traiter les peaux sensibles et/ou sèches, comprenant dans un support physiologiquement acceptable une quantité efficace d'au moins un microorganisme appartenant à l'espèce *Lactobacillus paracaseï* ou *caseï,* l'une de leurs fractions ou l'un de leurs métabolites, en association à une quantité efficace d'au moins un microorganisme appartenant à l'espèce *Bifidobacterium longum* ou *Bifidobacterium lactis,* l'une de leurs fractions ou l'un de leurs métabolites et comprenant en outre au moins un cation divalent.

La présente invention concerne selon encore un autre de ses aspects, une composition cosmétique et/ou dermatologique, notamment utile pour prévenir et/ou traiter les peaux sensibles et/ou sèches, comprenant dans un support physiologiquement acceptable une quantité efficace d'au moins un microorganisme appartenant à l'espèce *Lactobacillus paracaseï* ou *casei*; l'une de leurs fractions ou l'un de leurs métabolites, en association à une quantité efficace d'au moins un microorganisme appartenant à l'espèce *Bifidobacterium Longum* NCC 490 (CNCM I-2170) ou *Bifidobacterium lactis* NCC 2818 (CNCM I-3446), l'une de leurs fractions ou l'un de leurs métabolites.

La présente invention concerne également selon un autre de ses aspects, une composition pour l'absorption orale notamment de type complément alimentaire, comprenant dans un support physiologiquement acceptable une quantité efficace d'au moins un microorganisme appartenant à l'espèce *Lactobacillus paracaseï* ST11 NCC 2461, (CNCM I-2116), l'une de ses fractions ou l'un de ses métabolites en association à une quantité efficace d'au moins un microorganisme appartenant à l'espèce *Bifidobacterium longum* ou *Bifidobacterium lactis,* l'une de leurs fractions ou l'un de leurs métabolites.

La présente invention concerne selon un autre de ses aspects, une composition pour l'absorption orale notamment de type complément alimentaire, comprenant dans un support physiologiquement acceptable une quantité efficace d'au moins un microorganisme appartenant à l'espèce *Lactobacillus paracaseï* ou *caseï,* l'une de leurs fractions ou l'un de leurs métabolites, en association à une quantité efficace d'au moins un microorganisme appartenant à l'espèce *Bifidobacterium longum* ou *Bifidobacterium lactis,* l'une de leurs fractions ou l'un de leurs métabolites et comprenant en outre au moins un cation divalent.

La présente invention concerne selon encore un autre de ses aspects, une composition pour l'absorption orale notamment de type complément alimentaire, comprenant dans un support physiologiquement acceptable une quantité efficace d'au moins un microorganisme appartenant à l'espèce *Lactobacillus paracaseï* ou *caseï,* l'une de leurs fractions ou l'un de leurs métabolites en association à une quantité efficace d'au moins un microorganisme appartenant à l'espèce *Bifidobacterium longum* NCC 490 (CNCM I-2170) ou *Bifidobacterium lactis* NCC 2818 (CNCM I-3446), l'une de leurs fractions ou l'un de leurs métabolites.

### Peau sensible et/ou sèche

Comme précisé précédemment, une peau sensible est différente d'une peau allergique. Sa réactivité ne relève pas d'un processus immunologique et se traduit généralement uniquement par des sensations dysesthétiques.

Pour des raisons évidentes, l'absence de signes visibles rend difficile le diagnostic de peau sensible. Le plus souvent ce diagnostic repose sur l'interrogatoire du patient. Cette symptomatologie a en outre pour intérêt de permettre de différencier la peau sensible associée ou non à une peau sèche, de l'irritation ou de l'allergie de contact pour lesquelles il existe en revanche des signes inflammatoires visibles.

En conséquence, la mise au point de produits "peaux sensibles" a nécessité de disposer d'outils dévaluation de la réaction sensorielle de la peau. Les premiers outils se sont inspirés dès leur conception de la caractéristique essentielle des peaux sensibles à savoir présence de signes d'inconfort induits par une application topique. Ainsi, le "stinging test" à l'acide lactique a été le premier test proposé. Il est réalisé par relevé des sensations de picotements rapportées par un volontaire après application d'une solution d'acide lactique à 10 % sur les ailes du nez. Les sujets rapportant des sensations modérées ou fortes de picotements sont appelées "stingers" et considérés comme étant à peau sensible. En raison de cette sensibilité cutanée à l'application topique de produit, ces sujets sont alors sélectionnés pour tester des produits dits peaux sensibles. Plus récemment, pour activer spécifiquement les terminaisons nerveuses périphériques, impliquées dans l'inconfort et appelées nocicepteurs, récemment identifiées comme étant impliquées dans la peau sensible, de nouveaux tests ont été proposés qui utilisent précisément d'autres inducteurs d'inconfort comme la capsaïcine.

Ce second type de test, décrit dans la demande EP 1 374 913, constitue également un autre outil particulièrement utile pour le diagnostic de peaux sensibles.

Au sens de la présente invention, les peaux sensibles couvrent les peaux irritables et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température ou la laine.

D'une manière générale, ces deux types de peau peuvent être associés à une sécheresse cutanée avec ou sans dartres ou à une peau qui présente un érythème.

Comme précisé précédemment, la sécheresse cutanée est souvent associée à une baisse du taux d'hydratation cutanée, évalué par cornéométrie et à une altération de la fonction barrière, mesurée par la perte insensible en eau.

La peau sèche se manifeste essentiellement par une sensation de tiraillements et/ou de tension. Celle-ci est aussi rugueuse au toucher et apparaît couverte de squames. Lorsque la peau est légèrement sèche, ces squames sont abondantes mais peu visibles à l'oeil nu. Elles sont de moins en moins nombreuses mais de plus en plus visibles à l'oeil nu lorsque ce désordre s'aggrave.

L'origine de cette sécheresse cutanée peut être de type constitutionnel ou acquis.

Dans le cas de peau sèche acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, la peau peut devenir alors momentanément et localement sèche. Cela peut concerner tout type de peau, normal et même gras.

Dans le cas de la peau sèche constitutionnelle, on peut distinguer deux catégories : les peaux pathologiques et les peaux non pathologiques.

Les peaux sèches constitutionnelles pathologiques sont essentiellement représentées par la dermatite atopique et les ichthyoses. Elles sont quasiment indépendantes des conditions extérieures.

La dermatite atopique est décrite comme associée à un déficit dans le métabolisme des lipides du stratum corneum et notamment des céramides. Cette pathologie se présente sous la forme d'une xérose plus ou moins chronique concernant une grande étendue du corps, associée à des poussées inflammatoires et prurigineuses par plaques.

Les ichthyoses sont des pathologies caractérisées par un déficit génétique affectant le processus de kératinisation à différents stades. Elles se manifestent par une desquamation importante par plaques.

Les peaux sèches constitutionnelles non pathologiques sont des peaux sèches dont la sévérité peut dépendre des facteurs extérieurs déjà évoqués. Rentrent dans cette catégorie de peau, la peau sénile (caractérisée par une diminution générale du métabolisme cutané avec l'âge), la peau fragile (très sensible aux facteurs extérieurs et souvent accompagnée d'érythème et de rosacée) et la xérose vulgaire (d'origine génétique probable et se manifestant en priorité sur le visage, les membres et le dos des mains).

Les compositions et procédé selon l'invention, s'avèrent ainsi tout particulièrement efficaces pour prévenir et/ou traiter les peaux sensibles et/ou sèches et plus particulièrement les peaux dites réactives, irritables et/ou intolérantes, les peaux sèches acquises et/ou les peaux sèches constitutionnelles.

L'utilisation et les compositions selon l'invention mettent en oeuvre au moins un microorganisme appartenant à l'espèce *Lactobacillus paracaseï* ou *caseï,* en particulier, un microorganisme appartenant à l'espèce *Lactobacillus paracaseï,* et notamment un microorganisme appartenant à l'espèce *Lactobacillus paracaseï* ST11 NCC 2461.

Plus particulièrement, il s'agit du microorganisme *Lactobacillus paracaseï* ST11 NCC 2461 déposé suivant le traité de Budapest à l'Institut Pasteur (28 rue du Docteur Roux-75024 Paris Cédex 15) - sous la désignation CNCM I-2116, d'une de ses fractions ou d'un de ses métabolites.

En ce qui concerne le microorganisme appartenant à l'espèce *Bifidobacterium Longum,* il peut plus particulièrement être choisi parmi le *Bifidobacterium Longum* NCC 490 (CNCM I-2170). Une autre souche de Bifidobacterium longum est commercialisée sous l'appellation *Bb 536* par MORINAGA.

En ce qui concerne le microorganisme appartenant à l'espèce *Bifidobacterium lactis,* il peut plus particulièrement s'agir de l'espèce *Bifidobacterium lactis* NCC 2818 (CNCM I-3446).

Selon un mode de réalisation particulier, l'utilisation et les compositions selon l'invention mettent en oeuvre au moins le microorganisme *Lactobacillus paracaseï* ST11, NCC 2461 (CNCM I-2116) l'une de ses fractions ou l'un de ses métabolites et au moins le microorganisme *Bifidobacterium Longum* NCC 490 (CNCM I-2170), l'une de ses fractions ou l'un de ses métabolites.

Selon un autre mode de réalisation particulier, l'utilisation et les compositions selon l'invention mettent en oeuvre le microorganisme *Lactobacillus paracaseï* ST11 NCC 2461 (CNCM I-2116), l'une de ses fractions ou l'un de ses métabolites, et au moins le microorganisme *Bifidobacterium lactis* NCC 2818 (CNCM I-3446), l'une de ses fractions ou l'un de ses métabolites.

Au sens de l'invention, le terme "métabolite" désigne toute substance issue du métabolisme des microorganismes considérés selon l'invention et dotée également d'une efficacité pour le traitement des peaux sensibles et/ou sèches.

Au sens de l'invention, le terme "fraction" désigne plus particulièrement un fragment dudit microorganisme doté d'une efficacité pour le traitement des peaux sèches et/ou sensibles par analogie audit microorganisme entier.

Chacun des microorganismes et/ou métabolites et/ou fractions correspondant peut être formulé dans un support approprié à raison d'au moins 10³ ufc/g, en particulier à raison de 10⁵ à 10¹⁵ ufc/g et plus particulièrement à raison de 10⁷ à 10¹² ufc/g.

Selon une variante de l'invention, ils peuvent être associés dans un rapport en ufc *Lactobacillus paracaseï* ou *caseï*/*Bifidobacterium longum* ou *Lactobacillus paracaseï* ou *caseï*/*Bifidobacterium lactis* variant de 0,5 à 1,5, en particulier de 0,7 à 1,2 et plus particulièrement de l'ordre de 1.

Avantageusement, l'utilisation et les compositions selon l'invention mettent en oeuvre au moins lesdits microorganismes dans des quantités équivalentes et plus particulièrement à raison de 10¹⁰ ufc respectivement.

Ces microorganismes peuvent être formulés à l'état de poudres, c'est-à-dire sous une forme sèche, ou sous forme de suspensions ou de solutions.

Si nécessaire, ces microorganismes peuvent être formulés sous une forme encapsulée de manière à améliorer significativement leur durée de survie. Dans un tel cas, la présence d'une capsule peut en particulier retarder ou éviter la dégradation du microorganisme au niveau du tractus gastro-intestinal.

Ces microorganismes peuvent en outre contenir être associés à au moins un microorganisme appartenant à une espèce distincte, notamment une espèce de type probiotique et/ou l'une de ses fractions et/ou l'un de ses métabolites.

Au sens de la présente invention, on entend par "microorganisme probiotique", un microorganisme vivant qui, lorsqu'il est consommé en quantité adéquate, a un effet positif sur la santé de son hôte "joint FAO/WHO Expert Consultation on Evaluation of Health and Nutritional Properties of Probiotic in Food Including Powder Milk with Live Lactic Acid Bacteria, 6 octobre 2001", et qui peut en particulier améliorer l'équilibre microbien intestinal.

Ces microorganismes convenant à l'invention peuvent être choisis notamment parmi les ascomycetes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* et *Lactobacillus* et leurs mélanges.

Comme ascomycetes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*

Des exemples spécifiques de microorganismes probiotiques sont *Bifidobacterium bifidum, Bifidobacterium infantis, Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus rhamnosus (Lactobacillus GG), Lactobacillus sake, Lactococcus lactis, Streptococcus thermophilus, Staphylococccus carnosus,* et *Staphylococcus xylosus et leurs mélanges.*

Plus particulièrement, il s'agit de microorganismes probiotiques issus du groupe des bactéries lactiques, comme notamment les *Lactobacillus* et/ou les *Bifidobacterium.* A titre illustratif de ces bactéries lactiques, on peut plus particulièrement citer les *Lactobacillus johnsonii, Lactobacillus reuteri, Lactobacillus rhamnosus, Bifidobacterium bifidum, Bifidobacterium breve, Bifidobacterium animalis, Bifidobacterium lactis, Bifidobacterium longum, Bifidobacterium infantis, Bifidobacterium adolescentis* ou *Bifidobacterium pseudocatenulatum* et leurs mélanges.

Une souche de *Bifidobacterium lactis* peut être obtenue chez Hansen (Chr. Hansen A/S, 10-12 Boege Alle, P.O. Box 407, DK-2970 Hoersholm, Danemark) sous l'appellation *Bb 12.*

Les espèces convenant tout particulièrement sont les *Lactobacillusjohnsonii* et *Bifidobacterium adolescentis,* respectivement déposés suivant le traité de Budapest avec l'Institut Pasteur (28 rue du Docteur Roux, F-75024 Paris cedex 15) sous les désignations suivantes CNCM I-1225 et CNCM I-2168 et leurs mélanges.

Dans le cas d'une association d'au moins un microorganisme appartenant à l'espèce *Lactobacillus paracaseï* ou *caseï* à au moins un microorganisme appartenant à l'espèce *Bifidobacterium longum,* l'utilisation ou une composition selon l'invention peut également mettre en oeuvre au moins un microorganisme appartenant à l'espèce *Bifidobacterium lactis.*

De la même façon, dans le cas d'une association d'au moins un microorganisme appartenant à l'espèce *Lactobacillus paracaseï* ou *caseï* à au moins un microorganisme appartenant à l'espèce *Bifidobacterium lactis,* l'utilisation ou une composition selon l'invention peut également mettre en oeuvre au moins un microorganisme appartenant à l'espèce *Bifidobacterium longum.*

Ces microorganismes et/ou leurs fractions et/ou métabolites peuvent être formulés dans un support approprié dans une quantité d'au moins 10³ ufc/g, en particulier à des doses variant de 10⁵ à 10¹⁵ ufc/g, et plus particulièrement de 10⁷ à 10¹² ufc/g de support.

Les formulations exposées précédemment pour les microorganismes appartenant aux espèces constituant les associations plus particulièrement considérées selon l'invention à savoir *Lactobacillus paracaseï* ou *caseï*, *Bifidobacterium Longum* et/ou *Bifidobacteriul lactis* peuvent bien entendu être considérées pour les microorganismes précités.

D'une manière générale, les compositions selon l'invention et en particulier celles destinées à être administrées par voie orale peuvent comprendre pour les microorganismes vivants de 10³ à 10¹⁵ ufc/g, en particulier de 10⁵ à 10¹⁵ ufc/g et plus particulièrement de 10⁷ à 10¹² ufc/g de microorganismes par gramme de support ou à des doses équivalentes calculées pour les microorganismes inactifs ou morts ou pour des fractions de microorganisme ou pour des métabolites produits. Les compositions à application topique selon l'invention comprennent généralement de 10³ à 10¹² ufc/g, en particulier de 10⁵ à 10¹⁰ ufc/g et plus particulièrement de 10⁷ à 10⁹ ufc/g de microorganismes notamment probiotiques.

Lorsque la composition comprend des métabolites, les teneurs en métabolites dans les compositions correspondent sensiblement aux teneurs susceptibles d'être produites par 10³ à 10¹⁵ ufc, en particulier 10⁵ à 10¹⁵ ufc, et plus particulièrement 10⁷ à 10¹² ufc de microorganismes vivants par gramme de support.

Dans le cas particulier des compositions devant être administrées par voie orale, la concentration en chaque microorganisme et/ou fraction et/ou métabolite correspondant peut être ajustée de manière à correspondre à des doses (exprimées en équivalent de microorganisme) variant de 5.10⁵ à 10¹³ ufc/j et en particulier de 10⁸ à 10¹¹ ufc/j.

Le ou les microorganisme(s) peu(ven)t être inclus dans la composition selon l'invention sous une forme vivante, semi-active ou inactivée, morte.

Il(s) peu(ven)t également être inclus sous forme de fractions de composants cellulaires ou sous la forme de métabolites. Le ou le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Dans le cas particulier des compositions topiques, il peut être avantageux de mettre en oeuvre ces microorganismes sous forme inactivée voire morte.

Selon une variante de l'invention, l'utilisation et les compositions selon l'invention peuvent mettre en oeuvre en outre un ou plusieurs cation(s) minéral(aux) divalent(s).

Dans le cadre de la présente invention, on peut utiliser les cations minéraux divalents sous différentes formes. Le cation minéral divalent peut ainsi être sous la forme d'un sel minéral ou organique, anhydre ou hydraté ou d'un complexe chelaté.

Ces sels peuvent être par exemple des carbonates, des bicarbonates, des sulfates, des glycérophosphates, des chlorures, des nitrates, des acétates, des hydroxydes, des oxydes, des sels d'α-hydroxyacides (citrates, tartrates, lactates, malates) ou d'acides de fruits, ou encore des sels d'acides aminés (aspartate, arginate, fumarate) ou des sels d'acides gras (palmitate, oléate, caséinate, béhénate).

Selon un mode de réalisation particulier, le cation minéral divalent est choisi parmi le manganèse, le cuivre et/ou le zinc.

Selon un autre mode de réalisation particulier, le cation minéral divalent est un métal alcalino-terreux. Comme métal alcalino-terreux utilisable dans l'invention, on peut citer le baryum, le calcium, le magnésium, le strontium et/ou le béryllium.

Avantageusement, le cation minéral divalent et notamment métal alcalino-terreux est utilisé dans la présente invention sous forme de sel. En particulier, le sel peut être choisi parmi le nitrate de calcium, le nitrate de strontium, le gluconate de magnésium, le lactate de calcium, le gluconate de strontium, le lactate de magnésium, le chlorure de calcium, le chlorure de strontium, le chlorure de magnésium, le carbonate de calcium, le sulfate de strontium, le sulfate de magnésium, le glycérophosphate de calcium, le citrate de calcium, le citrate de magnésium, l'acétate de strontium, l'acétate de magnésium et leurs mélanges.

Selon un mode de réalisation particulièrement avantageux, on utilise au moins un cation minéral divalent choisi parmi les sels de citrate, chlorure, gluconate, sulfate, lactate et/ou acétate, de strontium, de calcium et/ou de magnésium et leurs mélanges.

Le cation minéral divalent peut aussi être utilisé sous la forme d'un complexe chélaté notamment à des protéines cristallisées ou ionisées.

Le cation minéral divalent peut encore être sous une forme spécifique stockée par un microorganisme, par exemple de type levure, à l'image des levures séléniées.

Ainsi, les cations peuvent être introduits tels quels dans les compositions selon l'invention ou par le biais d'un composé ou mélange de composé(s), connus pour contenir au moins l'un de ces cations en une concentration élevée. Par exemple, comme source de sels métalliques, on peut utiliser un extrait de plantes ou levures enrichis en cations. De même, le calcium peut par exemple être introduit par l'intermédiaire d'un extrait lacté.

La teneur en cation minéral divalent utilisée dans les compositions selon l'invention dépend bien entendu de la forme du cation considéré et peut être déterminée à l'aide de simples expériences de routine. Ces doses quotidiennes peuvent en particulier aller de 100 µg à 5 g, plus particulièrement de 1 mg à 2 g, voire de 10 mg à 1,3 g.

Dans les compositions destinées à une administration orale selon l'invention, la concentration en cation minéral divalent peut être ajustée de manière à correspondre à des doses variant de 1 à 3000 mg/jour et en particulier de 10 à 2000 mg/jour.

Les compositions selon l'invention sont généralement administrées par voie topique ou par voie orale.

Les compositions selon l'invention peuvent se présenter sous toutes les formes galéniques normalement utilisées selon la voie d'utilisation.

Le support peut être de nature diverse selon le type de composition considérée.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des aliments pour animaux en particulier domestiques, des comprimés ou tablettes, des suspensions de bactéries liquides, des suppléments oraux sous forme sèche et les suppléments oraux sous forme liquide.

En particulier, la composition selon l'invention peut être une composition alimentaire pour la consommation humaine. Il peut s'agir en particulier d'aliments complets nutritionnels, de boissons, d'eaux minérales, de soupes, de suppléments diététiques et d'aliments de remplacement, de barres nutritionnelles, de confiserie, de produits à base de lait ou à base de lait fermenté, de yaourts, de poudres à base de lait, de produits de nutrition entérale, de compositions pour enfants et/ou nourrissons, de produits à base de céréales ou de produits à base de céréales fermentées, de glaces, de chocolat, de café, de produits "culinaires" tels que de la mayonnaise, de la purée de tomate ou des assaisonnements pour salades. La composition selon l'invention peut également être destinée aux animaux.

En ce qui concerne plus particulièrement les produits cosmétiques, il peut s'agir de solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique.

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules ou solutions. En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Les actifs selon l'invention peuvent être formulés avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires sont connus dans ce domaine et ne font pas ici l'objet d'une description détaillée.

Bien entendu, les compositions orales selon l'invention peuvent contenir plusieurs autres actifs.

A titre d'actifs utilisables, on peut citer, les vitamines B3, B5, B6, B8, C, E, ou PP, les caroténoïdes, les curcuminoïdes et la niacine.

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des anthocyanines.

La composition comprend avantageusement au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes, de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Les compositions cosmétiques et/ou dermatologiques, plus particulièrement concernées par une application topique, peuvent se présenter notamment sous forme de solutions aqueuses, hydroalcooliques ou huileuses, de dispersions du type des solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, de gel aqueux ou anhydre, ou encore de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, ou des compositions contre les piqûres d'insectes.

Les compositions selon l'invention peuvent également consister en des préparations solides constituant des savons ou des pains de nettoyage.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, la composition cosmétique et/ou dermatologique de l'invention peut contenir également des adjuvants habituels dans le domaine cosmétique pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composés siliconés comme les huiles siliconées et par exemple les cyclométhicone et diméthicone, les cires, les résines et les gommes siliconées. Ces composés peuvent être fonctionnalisés ou non.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO® par la société HENKEL, le mélange de PEG-6/PEG-32/Glycol Stéarate vendu sous la dénomination de Tefose® 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (200E).

Comme solvants utilisables dans l'invention, on peut citer les alcools inférieurs, notamment l'éthanol et l'isopropanol, le propylène glycol.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305® par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Comme actifs hydrophiles, on peut utiliser les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Comme actifs lipophiles, on peut utiliser le rétinol (vitamine A) et ses dérivés, le tocophérol (vitamine E) et ses dérivés, les céramides, les huiles essentielles.

On peut, en outre, associer les actifs selon l'invention, à des agents actifs destinés notamment à la prévention et/ou au traitement des affections cutanées.

En outre, la composition de l'invention peut contenir de façon avantageuse une eau thermale et/ou minérale, notamment choisie parmi l'eau de Vittel, les eaux du bassin de Vichy et l'eau de la Roche Posay.

Le procédé de traitement cosmétique de l'invention peut être mis en oeuvre notamment en appliquant les compositions cosmétiques et/ou dermatologiques telles que définies ci-dessus, selon la technique d'utilisation habituelle de ces compositions. Par exemple : applications de crèmes, de gels, de sérums, de lotions, de laits de démaquillage ou de compositions après-solaires sur la peau ou sur les cheveux secs, application d'une lotion pour cheveux sur cheveux mouillés, de shampooings, ou encore application de dentifrice sur les gencives.

Le procédé cosmétique selon l'invention peut être mis en oeuvre par administration topique ou par une prise orale, journalière par exemple, de l'association selon l'invention qui peut être par exemple formulée sous forme de gélules, gels, lotions, dragées, émulsions, comprimés, capsules ou ampoules buvables, en quantité et nombre adéquats, selon leur forme, pour que les actifs soient administrés à raison de 5.10⁵ à 10¹³ ufc par jour, en particulier 10⁶ à 10¹¹ ufc par jour, en microorganismes ou à des doses équivalentes en microorganismes partiellement inactivés ou morts ou en fractions de microorganismes ou en métabolites produits.

Selon un autre mode de réalisation, l'administration est répétée jusqu'à ce que le cation minéral divalent soit administré à des doses de l'ordre de 1 à 3000 mg par jour, et en particulier de 10 à 2000 mg par jour.

Le procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme "entre...et..." incluent les bornes inférieure et supérieure précisées. Les ingrédients sont mélangés, avant leur mise en forme, dans l'ordre et dans des conditions facilement déterminées par l'homme de l'art.

Les exemples ci-après sont présentés à titre illustratif et non limitatif du domaine de l'invention.

### Exemples de compositions pour la voie orale

**Exemple 1 : Stick poudre**

| **Principe actif** | |
|---|---|
| *Lactobacillus paracaseï* ST11 NCC 2461 (CNCM I-2116) | 10¹⁰ ufc |
| *Bifidobacterium longum* NCC 490 (CNCM I-2170) | 10¹⁰ ufc |
| Citrate de calcium | 50 mg |
| | |

| **Excipient** | |
|---|---|
| Gomme de xanthane | 0,8 mg |
| Benzoate de sodium | 0,2 mg |
| Maltodextrine | qsp 30g |

On peut prendre un stick par jour.

**Exemple 2 : Stick poudre**

| **Principe actif** | |
|---|---|
| *Lactobacillus paracaseï* ST11 NCC 2461 (CNCM I-2116) | 10¹⁰ ufc |
| *Bifidobacterium longum* NCC 490 (CNCM I-2170) | 10¹⁰ ufc |
| Citrate de magnésium | 50 mg |
| | |

| **Excipient** | |
|---|---|
| Gomme de xanthane | 0,8 mg |
| Benzoate de sodium | 0,2 mg |
| Maltodextrine | qsp 30g |

On peut prendre un stick par jour.

**Exemple 3 : capsule**

| **Principe actif** | **mg/capsule** |
|---|---|
| *Lactobacillus paracaseï* ST11 NCC 2461 (CNCM I-2116) | 10 ufc |
| *Bifidobacterium longum* NCC 490 (CNCM I-2170) | 10⁸ ufc |
| Gluconate de magnésium | 150 |
| Vitamine C | 60 |
| Stéarate de magnésium | 0,02 |

On peut prendre une à trois de ces capsules par jour.

**Exemple 4 : formulation de type dragée**

| **Matières actives** | **mg/dragée** |
|---|---|
| Gluconate de magnésium | 50 |
| *Lactobacillus paracaseï* ST11 NCC 2461 (CNCM I-2116) | 5,10⁸ ufc |
| *Bifidobacterium longum* NCC 490 (CNCM I-2170) | 5,10⁸ ufc |
| Citrate de calcium | 200 |
| | |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

**Exemple 5 : formulation de type dragée**

| **Matières actives** | **mg/dragée** |
|---|---|
| Lactate de magnésium | 50 |
| *Bifidobacterium longum* NCC 490 (CNCM I-2170) | 10⁹ ufc |
| *Lactobacillus paracaseï* ST11 NCC 2461 (CNCM I-2116) | 10⁹ ufc |
| Lactate de calcium | 200 |
| | |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvinylidone | 6 |
| Dioxyde de titane | 0,3 |
| Agent de coloration | 5 |

Ce type de dragée peut être pris 1 à 3 fois par jour.

**Exemple 6 : Stick poudre**

| **Principe actif** | |
|---|---|
| *Lactobacillus paracaseï* ST11 NCC 2461 (CNCM I-2116) | 10¹⁰ ufc |
| *Bifidobacterium lactis* NCC 2818 (CNCM I-3446) | 10¹⁰ ufc |
| Citrate de calcium | 50 mg |
| | |

| **Excipient** | |
|---|---|
| Gomme de xanthane | 0,8 mg |
| Benzoate de sodium | 0,2 mg |
| Maltodextrine | qsp 30g |

On peut prendre un stick par jour.

**Exemple 7 : Stick poudre**

| **Principe actif** | |
|---|---|
| *Lactobacillus paracaseï* ST11 NCC 2461 (CNCM I-2116) | 10¹⁰ ufc |
| *Bifidobacterium lactis* NCC 2818 (CNCM I-3446) | 10¹⁰ ufc |
| Citrate de magnésium | 50 mg |
| | |

| **Excipient** | |
|---|---|
| Gomme de xanthane | 0,8 mg |
| Benzoate de sodium | 0,2 mg |
| Maltodextrine | qsp 30g |

On peut prendre un stick par jour.

**Exemple 8 : capsule**

| **Principe actif** | **mg/capsule** |
|---|---|
| *Lactobacillus paracaseï* ST11 NCC 2461 (CNCM I-2116) | 10 ufc |
| *Bifidobacterium lactis* NCC 2818 (CNCM I-2116) | 10⁸ ufc |
| Gluconate de magnésium | 150 |
| Vitamine C | 60 |
| Stéarate de magnésium | 0,02 |

On peut prendre une à trois de ces capsules par jour.

**Exemple 9 : formulation de type dragée**

| **Matières actives** | **mg/dragée** |
|---|---|
| Gluconate de magnésium | 50 |
| *Lactobacillus paracaseï* ST11 NCC 2461 (CNCM I-2116) | 5,10⁸ ufc |
| *Bifidobacterium lactis* NCC 2818 (CNCM I-3446) | 5,10⁸ ufc |
| Citrate de calcium | 200 |
| | |

| **Excipient du noyau de la dragée** | |
|---|---|
| Cellulose micro-cristalline | 70 |
| Encompress^{™} | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

**Exemple 10 : formulation de type dragée**

| **Matières actives** | **mg/dragée** |
|---|---|
| Lactate de magnésium | 50 |
| *Bifidobacterium lactis* NCC 2818 (CNCM I-3446) | 10⁹ ufc |
| *Lactobacillus paracaseï* ST11 NCC 2461 (CNCM I-2116) | 10⁹ ufc |
| Lactate de calcium | 200 |
| | |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvinylidone | 6 |
| Dioxyde de titane | 0,3 |
| Agent de coloration | 5 |

Ce type de dragée peut être pris 1 à 3 fois par jour.

### Exemples de composition pour la voie topique

**Exemple 11 : lotion pour le visage des peaux sensibles**

| | |
|---|---|
| Poudre de *Lactobacillus paracaseï* ST11 NCC 2461 (CNCM I-2116) | 5,00 |
| Poudre de *Bifidobacterium longum* NCC 490 (CNCM I-2170) | 5,00 |
| Gluconate de magnésium | 3,00 |
| Lactate de calcium | 2,00 |
| Antioxydant | 0,05 |
| Isopropanol | 40,0 |
| Conservateur | 0,30 |
| Eau | qsp 100% |

**Exemple 12 : lait pour le soin du visage des peaux sèches et sensibles**

| | |
|---|---|
| Chlorure de magnésium | 3,00 |
| Ascorbate de calcium | 3,00 |
| Poudre de *Lactobacillus paracaseï* ST11 NCC 2461 (CNCM I-2116) | 5,00 |
| Poudre de *Bifidobacterium longum* NCC 490 (CNCM I-2170) | 5,00 |
| Stéarate de glycérol | 1,00 |
| Alcool cétylstéarylique/alcool cétylstéarylique oxyéthyléné à 30 moles OE (Sinnowax AO® vendu par la société HENKEL) | 3,00 |
| Alcool cétylique | 1,00 |
| Diméthicone (DC 200 Fluid® vendu par la société DOW CORNING) | 1,00 |
| Huile de vaseline | 6,00 |
| Myristate disopropyle (Estol IMP 1514 vendu® par UNICHEMA) | 3,00 |
| Antioxydant | 0,05 |
| Glycérine | 20,00 |
| Conservateur | 0,30 |
| Eau | qsp 100 |

**Exemple 13 : gel pour le soin du visage des peaux sensibles**

| | |
|---|---|
| Nitrate de strontium | 4,00 |
| Poudre de *Lactobacillus paracaseï* ST11 NCC 2461 (CNCM I-2116) | 5,00 |
| Poudre de *Bifidobacterium longum* NCC 490 (CNCM I-2170) | 5,00 |
| Hydroxypropylcellulose (Klucel H® vendu par la société HERCULES) | 1,00 |
| Vitamine E | 2,50 |
| Antioxydant | 0,05 |
| Isopropanol | 40,00 |
| Conservateur | 0,30 |
| Eau | qsp 100 % |

**Exemple 14 : lait pour le soin du visage des peaux sèches et sensibles**

| | |
|---|---|
| Ascorbate de magnésium | 3,00 |
| Huile de pépin de cassis | 4,00 |
| Huile de bourrache | 4,00 |
| Poudre de *Lactobacillus paracaseï* ST11 NCC 2461 (CNCM I-2116) | 5,00 |
| Poudre de *Bifidobacterium longum* NCC490 (CNCM I-2170) | 5,00 |
| Stéarate de glycérol | 1,00 |
| Alcool cétylstéarylique/alcool cétylstéarylique oxyéthyléné à 3 moles OE (Sinnowax AO® vendu par la société HENKEL) | 3,00 |
| Alcool cétylique | 1,00 |
| Diméthicone (DC 200 Fluid® vendu par la société Dow Corning) | 1,00 |
| Huile de vaseline | 6,00 |
| Myristate disopropyle (Estol IPM 1514® vendu par la société Unichema) | 3,00 |
| Glycérine | 20,00 |
| Conservateur | 0,30 |
| Eau | qsp 100 |

**Exemple 15 : lotion pour le visage des peaux sensibles**

| | |
|---|---|
| Poudre de *Lactobacillus paracaseï* ST11 NCC 2461 (CNCM I-2116) | 5,00 |
| Poudre de *Bifidobacterium lactis* NCC 2818 (CNCM I-3446) | 5,00 |
| Gluconate de magnésium | 3,00 |
| Lactate de calcium | 2,00 |
| Antioxydant | 0,05 |
| Isopropanol | 40,0 |
| Conservateur | 0,30 |
| Eau | qsp 100 % |

**Exemple 16 : lait pour le soin du visage des peaux sèches et sensibles**

| | |
|---|---|
| Chlorure de magnésium | 3,00 |
| Ascorbate de calcium | 3,00 |
| Poudre de *Lactobacillus paracaseï* ST11 NCC 2461 (CNCM I-2116) | 5,00 |
| Poudre de *Bifidobacterium lactis* NCC2818 (CNCM I-3446) | 5,00 |
| Stéarate de glycérol | 1,00 |
| Alcool cétylstéarylique/alcool cétylstéarylique oxyéthyléné à 30 moles OE (Sinnowax AO® vendu par la société HENKEL) | 3,00 |
| Alcool cétylique | 1,00 |
| Diméthicone (DC 200 Fluid® vendu par la société DOW CORNING) | 1,00 |
| Huile de vaseline | 6,00 |
| Myristate d'isopropyle (Estol IMP 1514 vendu® par UNICHEMA) | 3,00 |
| Antioxydant | 0,05 |
| Glycérine | 20,00 |
| Conservateur | 0,30 |
| Eau | qsp 100 |

**Exemple 17 : gel pour le soin du visage des peaux sensibles**

| | |
|---|---|
| Nitrate de strontium | 4,00 |
| Poudre de *Lactobacillus paracaseï* ST11 NCC 2461 (CNCM I-2116) | 5,00 |
| Poudre de *Bifidobacterium lactis* NCC 2818 (CNCM I-3446) | 5,00 |
| Hydroxypropylcellulose (Klucel H® vendu par la société HERCULES) | 1,00 |
| Vitamine E | 2,50 |
| Antioxydant | 0,05 |
| Isopropanol | 40,00 |
| Conservateur | 0,30 |
| Eau | qsp 100 % |

**Exemple 18 : lait pour le soin du visage des peaux sèches et sensibles**

| | |
|---|---|
| Ascorbate de magnésium | 3,00 |
| Huile de pépin de cassis | 4,00 |
| Huile de bourrache | 4,00 |
| Poudre de *Lactobacillus paracaseï* ST11 NCC 2461 (CNCM I-2116) | 5,00 |
| Poudre de *Bifidobacterium lactis* NCC 2818(CNCM I-2446) | 5,00 |
| Stéarate de glycérol | 1,00 |
| Alcool cétylstéarylique/alcool cétylstéarylique oxyéthyléné à 3 moles OE (Sinnowax AO® vendu par la société HENKEL) | 3,00 |
| Alcool cétylique | 1,00 |
| Diméthicone (DC 200 Fluid® vendu par la société Dow Corning) | 1,00 |
| Huile de vaseline | 6,00 |
| Myristate d'isopropyle (Estol IPM 1514® vendu par la société Unichema) | 3,00 |
| Glycérine | 20,00 |
| Conservateur | 0,30 |
| Eau | qsp 100 |

### Exemple 19 : Etude d'efficacité

Une composition orale à base de microrganisme probiotique (B) a été testée pour son efficacité vis-à-vis de la sécheresse et sensibilité cutanée au regard d'une composition placebo (A). Leur composition est la suivante :
A : Maltodextrine
B : 1x10¹⁰ ufc *Lactobacillus paracaseï* ST11 NCC 2461 (CNCM I-2116) + 1x10¹⁰ ufc *Bifidobacterium lactis* NCC 2818 (CNCM I-3446).

Le traitement consiste à administrer quotidiennement et par voie orale une unique unité de traitement pendant une durée de huit semaines.

Cette étude a été réalisée sur 66 sujets adultes de sexe féminin dont l'âge est compris entre 18 et 50 ans, et qui ont été identifiés à la suite d'une évaluation clinique (score clinique de la sécheresse des jambes et de la rugosité du visage) et dune auto-évaluation par questionnaire (questionnaire peau sensible validée) comme des sujets à peaux sèches et sensible. Ces 66 sujets on été répartis en 2 groupes parallèles de 33 sujets, avec un groupe recevant le produit testé et un groupe recevant le placebo.

L'effet du complément testé est apprécié par comparaison à la formulation témoin dite placebo. Les résultats obtenus figurent dans le tableau I ci-après.

**Tableau I**

| **% de variation entre J1 et J57 et significativité *versus* placébo**^{**1**} | **Complément alimentaire à base de probiotiques selon l'invention (B)** |
|---|---|
| Score clinique : Diminution par rapport à J1 Rugosité du visage Sécheresse des jambes | -79 % (p=0,06) -60 % (p=0,02) |
| Auto-évaluation : Diminution par rapport à J1 Sécheresse des jambes Bioanalyse : Sensibilité cutanée | -28% (p=0,2) -60 % (p=0,02) |
| Facteur d'hydratation : Augmentation par rapport à J1 Urée Lactate de sodium | +28 % (p=0,6) Maintien du taux (p=0,15) alors que diminution de 60 % avec le placebo |

| | |
|---|---|
| ¹ : Analyse des contrastes entre J1 et J57 entre le groupe traité et le groupe placebo. | |

La composition orale conforme à l'invention de l'exemple a été testée en terme de sensibilité cutanée chez les sujets considérés pour l'étude (évaluation de la sensibilité cutanée par un test à l'acide lactique ou stinging test).

Il a ainsi été constaté une réduction de la sensibilité cutanée d'environ -60 % - p=0,02) entre J1 et J57 chez les sujets traités.

## Revendications

1. Utilisation d'une quantité efficace d'au moins un microorganisme appartenant à l'espèce *Lactobacillus paracaseï* ou *casei;* l'une de leurs fractions ou l'un de leurs métabolites en association à une quantité efficace d'au moins un microorganisme appartenant à l'espèce *Bifidobacterium longum* ou *Bifidobacterium lactis,* l'une de leurs fractions ou l'un de leurs métabolites, pour la fabrication d'une composition dermatologique destinée à traiter et/ou prévenir les peaux sensibles réactives associées ou non à une sécheresse cutanée.

2. Utilisation selon la revendication 1, **caractérisée en ce que** le microorganisme appartenant à l'espèce *Lactobacillus paracaseï* ou *caseï* est le microorganisme *Lactobacillus paracaseï* ST11 NCC 2461 (CNCM I-2116).

3. Utilisation selon la revendication 1 ou 2, **caractérisée en ce que** le microorganisme appartenant à l'espèce *Bifidobacterium lactis* est le microorganisme *Bifidobacterium lactis* NCC 2818 (CNCM I-3446).

4. Utilisation selon la revendication 1, 2 ou 3, **caractérisée en ce que** le microorganisme appartenant à l'espèce *Bifidobacterium Longum* est le microorganisme *Bifidobacterium Longum* NCC 490 (CNCM I-2170).

5. Utilisation selon l'une quelconque des revendications précédentes, **caractérisée en ce qu'**elle met en oeuvre au moins le microorganisme *Lactobacillus paracaseï* ST11 NCC 2461 (CNCM I-2116), l'une de ses fractions ou l'un de ses métabolites et au moins le microorganisme *Bifidobacterium Longum* NCC 490 (CNCM I-2170), l'une de ses fractions ou l'un de ses métabolites.

6. Utilisation selon l'une quelconque des revendications précédentes, **caractérisée en ce qu'**elle met en oeuvre au moins le microorganisme *Lactobacillus paracaseï* ST11 NCC 2461 (CNCM I-2116), l'une de ses fractions ou l'un de ses métabolites et au moins le microorganisme *Bifidobacterium lactis* NCC 2818 (CNCM I-3446), l'une de ses fractions ou l'un de ses métabolites.

7. Utilisation selon l'une quelconque des revendications précédentes, **caractérisée en ce que** lesdits microorganismes et/ou une de leurs fractions et/ou un de leurs métabolites sont formulés dans un support physiologiquement acceptable en une quantité équivalente à au moins 10³ ufc/g, notamment variant de 10⁵ à 10¹⁵ ufc/g, et en particulier de 10⁷ à 10¹² ufc/g de support.

8. Utilisation selon l'une quelconque des revendications précédentes, **caractérisée en ce que** lesdits microorganismes sont associés dans un rapport en ufc *Lactobacillus paracaseï* ou *caseï*/*Bifidobacterium longum* ou *Lactobacillus paracaseï* ou *caseï*/*Bifidobacterium lactis* variant de 0,5 à 1,5, en particulier de 0,7 à 1,2 et plus particulièrement de l'ordre de 1.

9. Utilisation selon l'une quelconque des revendications précédentes, **caractérisée en ce qu'**elle met en oeuvre en outre au moins un autre microorganisme notamment de type probiotique, et/ou l'une de ses fractions et/ou l'un de ses métabolites.

10. Utilisation selon la revendication précédente, **caractérisée en ce que** ledit microorganisme est choisi parmi les ascomycètes telles que *Saccharomyces, Yarrowia, Kluyveromyces, Torulaspora, Schizosaccharomyces pombe, Debaromyces, Candida, Pichia, Aspergillus et Penicillium,* les bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* et *Lactobacillus* et leurs mélanges.

11. Utilisation selon l'une quelconque des revendications 9 ou 10, **caractérisée en ce que** le microorganisme est choisi parmi les *Saccharomyces cereviseae, Yarrowia lipolitica, Kluyveromyces lactis, Torulaspora, Schizosaccharomyces pombe, Candida, Pichia, Bifidobacterium bifidum, Bifidobacterium breve, Bifidobacterium animalis, Bifidobacterium lactis, BIfidobacterium longum, Bifidobacterium infantis, Bifidobacterium adolescentis, Bifidobacterium pseudocatenulatum, Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus rhamnosus (Lactobacillus GG), Lactobacillus sake, Lactococcus lactis, Streptococcus thermophilus, Staphylococccus carnosus et Staphylococcus xylosus* et leurs mélanges.

12. Utilisation selon l'une quelconque des revendications 9 à 11, **caractérisée en ce que** le microorganisme est issu du groupe des bactéries lactiques.

13. Utilisation selon l'une quelconque des revendications 9 à 12, **caractérisée en ce que** le microorganisme est choisi parmi les *Lactobacillusjohnsonii* (CNCM I-1225), *Bifidobacterium adolescentis* (CNCM I-2168) et leurs mélanges.

14. Utilisation selon l'une quelconque des revendications précédentes, **caractérisée en ce qu'**elle met en oeuvre en outre au moins un cation minéral divalent.

15. Utilisation selon la revendication 14, **caractérisée en ce que** ledit cation minéral divalent est un métal alcalino-terreux.

16. Utilisation selon la revendication 15, **caractérisée en ce que** ledit métal alcalino-terreux est choisi parmi le baryum, le calcium, le magnésium, le strontium, le béryllium et leurs mélanges.

17. Utilisation selon la revendication 16, **caractérisée en ce que** ledit cation est sous la forme d'un sel choisi parmi des carbonates, des bicarbonates, des sulfates, des glycérophosphates, des chlorures, des nitrates, des acétates, des hydroxydes, des oxydes, des sels d'α-hydroxyacides comme les citrates, tartrates, lactates et malates ou d'acides de fruits, des sels d'acides aminés comme les aspartates, arginates, et fumarates ou des sels d'acides gras comme les palmitates, oléates, caséinates et béhénates.

18. Utilisation selon l'une quelconque des revendications précédentes, **caractérisée en ce qu'**il s'agit d'une composition cosmétique.

19. Utilisation selon l'une quelconque des revendications 1 à 17, **caractérisée en ce qu'**il s'agit d'une composition alimentaire, et en particulier destinée à la consommation humaine.

20. Utilisation selon l'une quelconque des revendications 1 à 17, **caractérisée en ce qu'**il s'agit d'une composition dermatologique.

21. Procédé de traitement cosmétique pour prévenir et/ou traiter les peaux sensibles associées ou non à la peau sèche, comprenant l'administration, orale ou topique d'au moins une quantité efficace d'au moins un microorganisme appartenant à l'espèce *Lactobacillus paracaseï* ou *caseï,* l'une de leurs fractions ou l'un de leurs métabolites en association à une quantité efficace d'au moins un microorganisme appartenant à l'espèce *Bifidobacterium longum* ou *Bifidobacterium lactis* l'une de leurs fractions ou l'un de leurs métabolites.

22. Procédé selon la revendication 21, **caractérisé en ce que** lesdits microorganismes sont tels que définis en revendications 2 à 8.

23. Procédé selon la revendication 21 ou 22, **caractérisé en ce que** lesdits microorganismes et/ou fractions et/ou métabolites sont respectivement administrés à raison de 5.10⁵ à 10¹³ ufc par jour et en particulier 10⁸ à 10¹¹ ufc par jour.

24. Composition cosmétique et/ou dermatologique, notamment utile pour prévenir et/ou traiter les peaux sensibles et/ou sèches, comprenant dans un support physiologiquement acceptable une quantité efficace d'au moins un microorganisme appartenant à l'espèce *Lactobacillus paracasei* ST11 NCC 2461 (CNCM I-2116), l'une de ses fractions ou l'un de ses métabolites en association à une quantité efficace d'au moins un microorganisme appartenant à l'espèce *Bifidobacterium longum,* ou *Bifidobacterium lactis,* l'une de leurs fractions ou l'un de leurs métabolites.

25. Composition cosmétique et/ou dermatologique, notamment utile pour prévenir et/ou traiter les peaux sensibles et/ou sèches, comprenant dans un support physiologiquement acceptable une quantité efficace d'au moins un microorganisme appartenant à l'espèce *Lactobacillus paracaseï* ou *caseï,* l'une de leurs fractions ou l'un de leurs métabolites en association à une quantité efficace d'au moins un microorganisme appartenant à l'espèce *Bifidobacterium longum* ou *bifidobacterium lactis,* l'une de leurs fractions ou l'un de leurs métabolites et comprenant en outre au moins un cation minéral divalent.

26. Composition cosmétique et/ou dermatologique, notamment utile pour prévenir et/ou traiter les peaux sensibles et/ou sèches, comprenant dans un support physiologiquement acceptable une quantité efficace d'au moins un microorganisme appartenant à l'espèce *Lactobacillus paracaseï* ou *caseï,* l'une de leurs fractions ou l'un de leurs métabolites en association à une quantité efficace d'au moins un microorganisme appartenant à l'espèce *Bifidobacterium longum* NCC 490 (CNCM I-2170) ou *Bifidobacterium lactis* NCC 2818 (CNCM I-3446).

27. Composition pour l'absorption orale comprenant dans un support physiologiquement acceptable une quantité efficace d'au moins un microorganisme appartenant à l'espèce *Lactobacillus paracaseï* ST11 NCC 2461 (CNCM I-2116), l'un de ses fractions ou l'un de ses métabolites en association à une quantité efficace d'au moins un microorganisme appartenant à l'espèce *Bifidobacterium longum* ou *Bifidobacterium lactis,* l'une de leurs fractions ou l'un de leurs métabolites.

28. Composition pour l'absorption orale comprenant dans un support physiologiquement acceptable une quantité efficace d'au moins un microorganisme appartenant à l'espèce *Lactobacillus paracaseï* ou *casei;* l'une de leurs fractions ou l'un de leurs métabolites en association à une quantité efficace d'au moins un microorganisme appartenant à l'espèce *Bifidobacterium longum* ou *Bifidobacterium lactis,* l'une de leurs fractions ou l'un de leurs métabolites et comprenant en outre au moins un cation minéral divalent.

29. Composition pour l'absorption orale comprenant dans un support physiologiquement acceptable une quantité efficace d'au moins un microorganisme appartenant à l'espèce *Lactobacillus pracaseï* ou *caseï*, l'une de leurs fractions ou l'un de leurs métabolites en association à une quantité efficace d'au moins un microorganisme appartenant à l'espèce *Bifidobacterium longum* NCC 490 (CNCM I-2170) ou *Bifidobacterium lactis* NCC 2818 (CNCM I-3446).

30. Composition selon l'une quelconque des revendications 24 à 29, **caractérisée en ce que** lesdits microorganismes sont tels que définis en revendications 2 à 8.

31. Composition selon l'une quelconque des revendications 24, 26, 27, 29 et 30, **caractérisée en ce qu'**elle comprend en outre au moins un cation minéral divalent.

32. Composition selon la revendication 25, 28 ou 31, **caractérisée en ce que** ledit cation minéral divalent est tel que défmi en revendication 14 à 17.

33. Composition selon l'une quelconque des revendications 24 à 32, **caractérisée en ce qu'**elle contient en outre au moins un microorganisme tel que défini en revendication 9 à 13.
